(19) Europäisches Patentamt
European Patent Office
Office européen des brevets

(11) EP 0 845 453 A2

(12) **EUROPEAN PATENT APPLICATION**

(43) Date of publication:
**03.06.1998 Bulletin 1998/23**

(51) Int. Cl.$^6$: **C07C 67/055**, C07C 69/15

(21) Application number: **98102093.6**

(22) Date of filing: **06.02.1998**

(84) Designated Contracting States:
**AT BE CH DE DK ES FI FR GB GR IE IT LI LU MC NL PT SE**
Designated Extension States:
**AL LT LV MK RO SI**

(30) Priority: **13.05.1997 US 855163**

(71) Applicant:
**Celanese International Corporation**
**Dallas, Texas 75234 (US)**

(72) Inventor: **Anderson, John Eldon**
**Corpus Christi, TX 78413 (US)**

(74) Representative:
**Ackermann, Joachim, Dr.**
**Hoechst Research & Technology Deutschland GmbH & Co.KG,**
**Patent- und Lizenzabteilung,**
**Gebäude K 801**
**65926 Frankfurt am Main (DE)**

(54) **Safety system**

(57) The invention relates to a process for operating a vinyl acetate plant, in which plant gaseous oxygen, acetic acid, and ethylene are fed to a reactor, having an inlet and an outlet, to react therein to form vinyl acetate and water, wherein the unreacted gases from the outlet of the reactor are recycled to the inlet of the reactor and wherein a flammable limit is used as a shut-down signal for the process characterized in that the flammable limit is equal to the maximum concentration of oxygen which can safely comprise the inlet gas composition.

Fig. VA UNIT REACTOR/RECYCLE LOOP

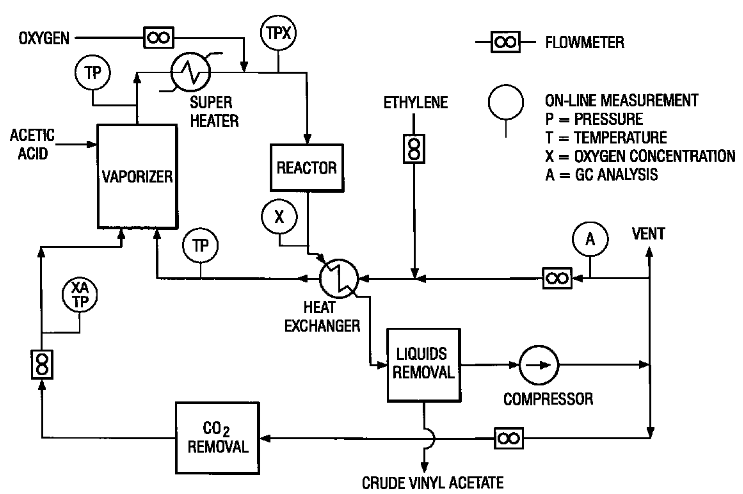


EP 0 845 453 A2

## Description

The invention relates to a process for operating a vinyl acetate plant, in which plant gaseous oxygen, acetic acid, and ethylene are fed to a reactor, having an inlet and an outlet, to react therein to form vinyl acetate and water, wherein the unreacted gases from the outlet of the reactor are recycled to the inlet of the reactor and wherein a flammable limit is used as a shutdown signal for the process. Of particular importance is the use of the system to effect the shut down of the plant in the case of a predicted flame front occurring at the reactor inlet. The process involves identifying the critical sites in the recycle system to predict the operation of the reactor.

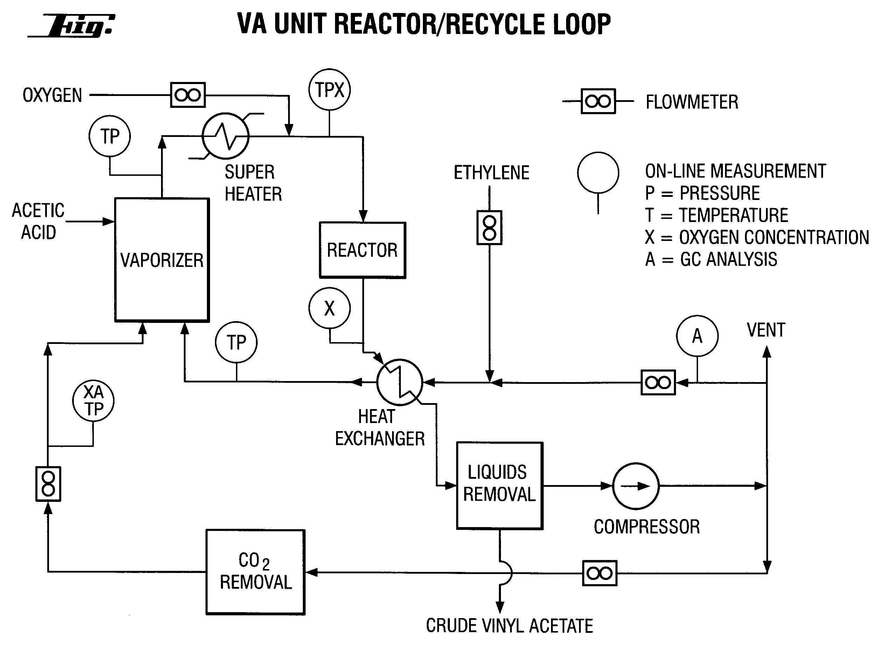

Vinyl acetate (VA) is traditionally manufactured by the reaction of acetic acid oxygen, and ethylene in the presence of a catalyst comprised of palladium and gold or palladium and cadmium on a solid support. The figure describing the invention contains a simplified flow diagram for the reactor/recycle loop of a typical VA unit. A mixture of gases (mostly ethylene plus oxygen, carbon dioxide and inerts) is discharged from the recycle compressor. Makeup ethylene is added to the recycle gas which is then heated and fed to a vaporizer tower. In the vaporiser the gas is saturated with acetic acid vapor. After leaving the vaporizer the gas is heated, fresh oxygen is added to it and it becomes the feed gas to the reactor. All of this is in preparation for the reaction:

$$\text{oxygen} + \text{acetic acid} + \text{ethylene} \rightarrow \text{vinyl acetate} + \text{water}$$

After leaving the reactor, the outlet stream is cooled and vinyl acetate, acetic acid, and water are condensed and removed. The dry gas is then compressed and recycled. The reaction is most efficiently run at as high an oxygen concentration as is possible without the oxygen concentration exceeding the flammable limit of the mixture. The flammable limit is defined as the lowest concentration of oxygen that will sustain combustion. If the oxygen concentration does exceed the flammable limit, a fire or an explosion could occur in the unit. Thus a major safety concern for a vinyl acetate (VA) unit is that the oxygen content of the feed to the reactor never exceeds the flammable limit of the gas. A safety system is normally employed to shut the unit down before the flammable limit is violated and the unit is put at risk.

An empirical equation for the flammable limit (FL) of a typical VA unit feed stream has been developed. The equation expresses the FL as a function of the temperature (T), pressure (P) and composition of the gas. FL has the units of per cent oxygen.

$$FL = f(T,\ P,\ \text{composition})$$

Approach to the flammable limit (AFL) is defined as the difference between the flammable limit of the gas and its oxygen content ($0_2$). It also has the units of per cent oxygen

$$AFL = FL - O_2$$

Vinyl acetate plants have long used the concept of a minimum AFL in designing the safety systems for a VA unit. The figure is a diagram of what comprises the minimum safe AFL and the target AFL for the plant. At AFL = 0, the oxygen in the reactor feed is equal to the flammable limit. Two safety factors combine to determine the minimum AFL. The first safety factor (SF-equation) allows for the uncertainty or error in the curve fit of the data which is used to establish the flammable limit equation. In practice, SF-equation is set equal to the value of the 95% confidence limit of the equation. The second safety factor (SF-instrument) is for instrument error. It allows for the uncertainty in the calculated AFL due to the instrument error in the measurements which are used in the calculations of the flammable limit and the AFL of the reactor feed gas. The sum of the two safety factors is the total uncertainty in the calculated value of the AFL. The true value of AFL (AFL-true) will fall within a band that is +/- the uncertainty around the calculated AFL (AFL-calc). The part of the band of most concern is its lower limit where the possible true AFL has its smallest value or

$$\text{AFL-true} = \text{AFL-calc} - \text{Uncertainty}$$

This means that the reactor feed could be in the flammable region if the calculated AFL is less than the minimum AFL. Steps must be taken before the calculated AFL reaches the minimum AFL. The unit must be shut down if necessary.

A third safety factor (SF-upset) is added to the minimum AFL to determine the target AFL as illustrated in the figure. The target AFL is the value of AFL at which the plant has chosen to run. It is the AFL in the operating instructions to the operators. SF-upset is for small upsets, those normal variations and disturbances always present in unit operations. The safety shutdown system is designed to remove the oxygen feed from the unit if the operating (calculated) AFL reaches the minimum AFL or slightly above it. Therefore the SF-upset must be larger than the changes in AFL which are caused by the normal variations in operating variables. Otherwise the unit will shut down too frequently, However SF-upset needs to be reasonably small because production efficiency and rates improve as the oxygen content of the reactor feed approaches its flammable limit and AFL decreases. Sizing SF-upset becomes a balance between production efficiency and the frequency of nuisance shutdowns.

Thus an important design objective is a shutdown system which will allow the plant to operate both safely and close to the minimum AFL. The obvious solution is to monitor the flammable limit and the oxygen concentration of the reactor feed and shut down when their difference decreases to the minimum. However in the past there have been two problems with this approach.

1. Flammable limit is not a measurable quantity. It must be calculated using an empirical equation such as the one shown above and the appropriate plant measurements. The problem becomes one of reliability of the data collection and computing equipment. Only in recent years has it been felt that process computers had the reliability for use in a safety shutdown system.

2. The oxygen concentration measurement is not a real-time value. The value from an online oxygen analyzer runs 30 to 40 seconds behind the actual oxygen concentration in the feed. The delay is due to the deadtime in the sample handling system of the analyzer. This means that if a sudden change in the oxygen concentration moves the reactor feed into the flammable region, it will be 30 to 40 seconds before the analyzer reports the change to the safety system and any action is taken. Even though going into the flammable region does not necessarily mean that a fire will occur, the 30 second window of opportunity for a fire is an unacceptable risk.

In the past, the two above problems had been avoided by not using a calculated online AFL for shut down, but by basing the safety system on an inferred AFL. Rather than limiting the AFL directly, AFL was limited indirectly by limiting the flows, temperatures, and pressures which determine AFL. Instruments for these variables do give values in real-time. Thus a target set of conditions was chosen which gave a safe AFL. The deviations of the flows, temperatures, and pressures from their target values were then limited by shutdown initiators. Each variable was limited so that its maximum deviation (from its target value to its shutdown value) would not cause the AFL to move from target AFL to the minimum AFL. Since this safety system did not calculate AFL on-line, it could not determine the combined effect on AFL of several variables moving simultaneously. As a result, and in order to allow for two variables moving at the same time, the SF-upset was sized to be equal to the sum of the two largest of the possible single variable AFL deviations. If Δ AFL is defined as the largest possible change in AFL due to a single operating variable going to its shutdown value, then

$$\text{SF-upset} \cong 2 * \Delta \text{ AFL}$$

It was therefore an object of the invention to provide a process as mentioned at the beginning which overcomes the problems described above.

This object has been solved by a process mentioned at the beginning which is characterized in that the flammable limit is equal to the maximum concentration of oxygen which can safely comprise the inlet gas composition.

Therefore, subject matter of the invention is a process as set out in claim 1. Preferred embodiments are given in the dependent claims.

The present invention describes and claims an improved safety shutdown system using an on-line AFL procedure. It monitors the AFL of the reactor feed and directly limits its deviation from target. Since this system monitors AFL directly, the safety system sees and protects against the effect of several operating variables moving at the same time, SF-upset need only allow the normal deviation in AFL, the largest of which is Δ AFL.

$$\text{SF-upset} \cong \Delta \text{ AFL}$$

The two problems with the on-line method mentioned above are now solved by the method of this invention.

1. Flammable limit of the reactor feed is calculated continuously and on a real-time basis. It is the maximum concentration of oxygen which is possible at the moment under safe operating conditions. Triple redundant process computers do the data collection and computations and have the required reliability for use in a safety system. The composition of the dry gas at the reactor inlet is determined by material balance from measurements of upstream flows and compositions. Finally an on-line flammable limit of the reactor feed is calculated using the experimentally derived equation for flammable limit.

2. The delay in the oxygen analysis is caused to be acceptable by calculating a predicted oxygen in the reactor feed. The transportation time of the gas flow from the outlet of the reactor back to the inlet of the reactor is significantly larger than the delay in the oxygen analyzers. Starting with the measurement of the oxygen concentration at the outlet of the reactor, the oxygen at the reactor feed can be calculated by material balance using the flows and oxygen concentrations of the added flows (makeup ethylene, outlet of the $CO_2$ removal system, and the fresh oxygen feed).

The figure identifies the various measurements necessary to carry out the best mode of this invention. On a dry basis (no water, acetic acid or vinyl acetate) the oxygen concentration measured at the reactor outlet and at the discharge of the recycle compressor are essentially the same. The important point is that even though the real time measurement of oxygen concentration at the reactor outlet is as much as 40 seconds delayed, this measurement of the oxygen concentration is available for use in predicting the oxygen concentration at the reactor inlet prior to the gas actually arriving at the reactor inlet. This gives the safety system time to respond and take action.

The value for the predicted oxygen needs to be accurate within 0. 1 % oxygen in order to have the desired close control of AFL. Oxygen analyzers are capable of that accuracy, but directly calculating an oxygen concentration to that accuracy by material balance using flows from field instruments is not practical. The required accuracy is achieved by adding a moving bias to the material balance equation to generate the predicted oxygen. The bias is a slow moving updated value and its purpose is to force the average of predicted oxygen to equal the average of measured oxygen at the reactor feed. The equations for the predicted oxygen are:

$$FFD = FRG + FET + FCO + FO_2$$

$$BI = FFD*XIFDm - (FRG*XRX + FCO*XCO + FO_2*100)$$

$$BIAS = \text{first order lag ( BI )}$$

$$XFDP = (FRG*XRX + FCO*XCO + FO_2*100 + BIAS)/FFD$$

where:

| | |
|---|---|
| BI | -- instantaneous bias, scfm |
| BIAS | -- average bias, scfm |
| FCO | -- flow of $CO_2$ removal outlet, scfm |
| FET | -- flow of ethylene feed, scfm |
| FFD | -- flow of reactor feed (dry basis), scfm |
| $FO_2$ | -- flow of oxygen feed, scfm |
| FRG | -- flow of recycle gas, scfm |
| XCO | -- oxygen conc. of $CO_2$ removal outlet, mol % |
| XFDm | -- oxygen conc. of reactor feed (measured), mol % |
| XFDP | -- oxygen conc. of reactor feed (predicted), mol % |
| XRX | -- oxygen conc. of reactor outlet, mol % |

Thus, the predicted oxygen combines the accuracy of the analyzer with the real-time response of the material balance calculations. The time constant of the averaging first order lag needs to be long enough to create a slow moving bias as compared to the 30 to 40 second delay in the oxygen analyzers.

The bias is the error in the closure of the oxygen material balance from reactor outlet to reactor inlet. All measurement errors are lumped into that one number. In addition to its usefulness as an on-line measuring tool, it is an excellent indicator of the status of the instruments used in the material balance.

Thus, according to the method of this invention, a recycle loop of reactants is maintained safely below the flammable limit in the system wherein measurements are made at specific points in the loop, product is removed in a liquids removal system, and reactants are added as indicated in the figure. In the best mode, beginning at the acetic acid feed point in the loop, the recycle gases are saturated with acetic acid in a vaporizer, the mixture is heated in a superheater, oxygen is added, and the gas stream is carried to the reactor. After reaction, the reactants and product are cooled by contact with a portion of the recycle stream gases, the condensed product is removed, and the recycle gases are compressed and split; a portion being used to cool the reactor outlet stream, and a portion used to remove by-product carbon dioxide. Measurements critical to the flammable limit are made as follows, (1) oxygen concentration at the reactor outlet, the point of analysis is furthest from the reactor inlet to allow more time for the instrument lag inherent in the system as disclosed hereinabove, (2) temperature and pressure after the in-process heat exchanger, since the temperature of the gas stream has been raised in the heat exchanger, (3) temperature and pressure after the $CO_2$ removal system, and (4) temperature and pressure after oxygen addition. Each of these measurements is taken immediately after a process point which triggers a modification in the gas stream affecting the FL.

The most useful advantage of the method of this invention is in being able to shut off the ingress of fresh oxygen to the recycle stream as an effective shutdown system. As the measured parameters of the system predict a flammable mixture at the reactor inlet, the oxygen can be shut down, thus, in effect, quenching the reaction.

## Claims

1. A process for operating a vinyl acetate plant, in which plant gaseous oxygen, acetic acid, and ethylene are fed to a reactor, having an inlet and an outlet, to react therein to form vinyl acetate and water, wherein the unreacted gases from the outlet of the reactor are recycled to the inlet of the reactor and wherein a flammable limit is used as a shutdown signal for the process characterized in that the flammable limit is equal to the maximum concentration of oxygen which can safely comprise the inlet gas composition.

2. A process as claimed in Claim 1, wherein the flammable limit is calculated from signals generated by measurement of temperature and pressure at the inlet of the reactor and a derived value for the inlet gas composition.

3. A process as claimed in Claim 2, wherein the inlet gas composition is calculated from the gas composition upstream of the reactor inlet.

4. A process as claimed in Claim 2, wherein the inlet gas composition is calculated from the gas composition downstream of the reactor outlet.

5. A process as claimed in Claim 3, wherein the inlet gas composition is calculated using material balance equations and measurements of flow and composition.

6. A process as claimed in Claim 4, wherein the inlet gas composition is calculated using material balance equations and measurements of flow and composition.

7. A process as claimed in Claim 2, wherein a component of the inlet gas composition is the oxygen concentration.

8. A process as claimed in Claim 2, wherein the derived value for the inlet gas composition is influenced by adding a bias factor which is a function of the measured oxygen concentration of the inlet gas composition.

Fig.

VA UNIT REACTOR/RECYCLE LOOP